# FASCICULE DE BREVET EUROPEEN

(11) **EP 3 752 129 B1**
(45) Date de publication et mention de la délivrance du brevet: **24.11.2021**
(21) Numéro de dépôt: 19710750.1
(22) Date de dépôt: 15.02.2019
(51) Int. Cl.: A61K 9/08, A61K 47/10, A61K 47/18, A61K 31/64, A61P 9/12

(54) **COMPOSITION MULTIUSAGE DE TORASEMIDE**
MEHRZWECK-TORASEMID-ZUSAMMENSETZUNG
MULTI-USE TORASEMIDE COMPOSITION

(30) Priorité: 16.02.2018 FR 1851353
(43) Date de publication de la demande: 23.12.2020
(73) Titulaire: Vetoquinol SA, 70200 Magny-Vernois (FR)
(72) Inventeur: MOREAU, Marinette, 70200 SAINT-GERMAIN (FR); LEGO, Elodie, 70200 LURE (FR)
(74) Mandataire: Novagraaf Technologies
(86) Numéro de dépôt international: PCT/FR2019/050345
(87) Numéro de publication internationale: WO 2019/158873

(56) Documents cités:
- US-A- 4 861 786
- US-A1- 2017 000 814

## Description

### Domaine technique

La présente invention se rapporte à une composition aqueuse comprenant du torasémide et au moins un solvant organique, à un flacon ou récipient comprenant ladite composition et à un kit comprenant ledit flacon ou récipient et un système d'administration de la composition (par exemple une seringue).

L'invention concerne également une utilisation d'au moins un solvant organique pour augmenter la stabilité et/ou les propriétés antimicrobiennes d'une composition comprenant du torasémide.

Enfin, la présente invention vise aussi un procédé de préparation de composition selon l'invention.

### Etat de la technique

Le torasémide ou torsémide (1-Isopropyl-3-[(4-m-toluidino-3-pyridyl)sulfonyl]urea est un principe actif connu, qui possède une forte action diurétique. Il est utilisé dans le traitement des œdèmes associés à des insuffisances cardiaques, des maladies rénales et en traitement de l'hypertension, en thérapeutique humaine et vétérinaire.

Le torasémide (C₁₆H₂₀N₄O₃S, M = 348,4 g.mol⁻¹, CAS : 56211-40-6) a pour formule développée la formule I suivante:

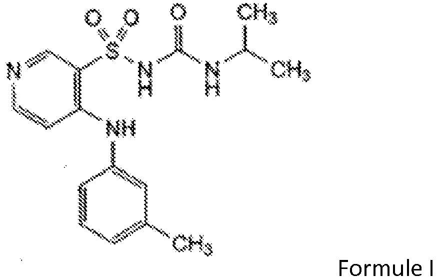

Il est commercialisé notamment sous les marques Demadex®, Diuver®, Dytor®, Examide® et Upcard ®.

Les maladies pour lesquelles un traitement comprenant du torasémide est préconisé, sont des maladies chroniques qui nécessitent généralement un traitement quotidien.

Des études récentes **([1])** ont recommandé de privilégier le torasémide pour un meilleur effet diurétique. Ainsi, l'intérêt pour le torasémide est croissant dans le traitement des affections cardiaques en particulier. En dépit de ces résultats encourageants, le torasémide a une marge thérapeutique faible (ou étroite), ce qui signifie que toute variation de sa concentration dans l'organisme, même faible ou modérée, peut éventuellement entraîner des effets indésirables, potentiellement graves. Il est donc important de pouvoir distribuer la dose requise.

Un déséquilibre ionique chez l'homme ou l'animal traité est observé avec un traitement chronique. Ce déséquilibre entraine d'autres effets indésirables tels que chute de tension ou augmentation de l'aldostérone et de la créatinine.

A ce jour, le furosémide (CAS : 54-31-9) constitue le traitement de référence. Il est généralement administré par voie orale ou intraveineuse.

US 2017/000814 A1 décrit une composition comprenant du furosémide.

Récemment, des comprimés sécables à base de torasémide ont été commercialisés pour répondre à la problématique de marge thérapeutique étroite et d'ajustement de la dose en fonction du poids de l'homme ou de l'animal.

Cependant cette solution ne permet pas d'ajuster la dose en tenant compte de toute la gamme de poids disponibles.

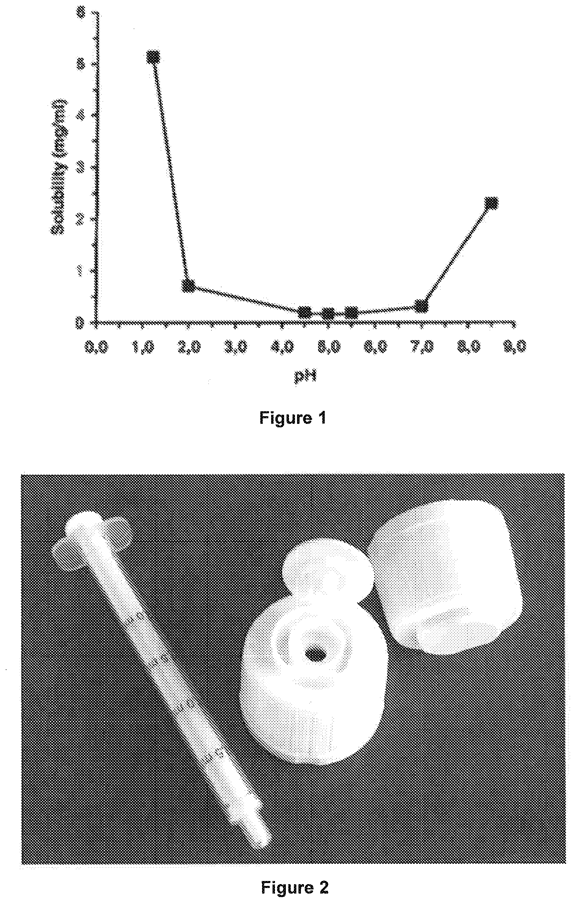

Par ailleurs, la solubilité du torasémide dépend du pH de la solution. La stabilité du torasémide en solution est en outre fonction des additifs et excipients employés dans la composition. Le torasémide n'est ainsi pas soluble à un pH compris entre 2 et 7 environ (figure 1).

Le torasémide est ainsi connu pour sa faible stabilité en milieu aqueux. On connait dans l'état de la technique des solutions alcalines injectables à usage unique à base de torasémide. Le document US 4,861,786 **([2])** en particulier décrit une composition alcaline injectable de torasémide comprenant un tampon compatible physiologiquement avec une valeur de pH comprise entre 9,3 et 9,9 et de 5 à 20 % d'un solvant organique, ledit solvant organique étant choisi dans le groupe comprenant les polyéthylènes glycols (ayant un poids moléculaire compris entre 100 et 1500 g.mol⁻¹), les polypropylènes glycols (ayant un poids moléculaire compris entre 50 et 1000 g.mol⁻¹), le glycérol, le propylène glycol, l'éthanol et le propanol. Ces compositions comprennent de 2 à 40 mg.ml⁻¹ de torasémide. Pour des raisons de stabilité, et en particulier l'apparition de cristaux, le pH doit être compris entre 9,3 et 9,9, et le pourcentage de solvants doit être inférieur à 20%.

Les compositions de torasémide alcalines injectables de l'état de la technique, ne présentent ainsi ni une stabilité suffisante, ni un effet antimicrobien garantissant leur bonne conservation. En outre, leurs propriétés ne permettent pas un usage multiple (ou multiusage), à savoir une utilisation répétée de la même composition sur une période de temps s'étalant de plusieurs heures à plusieurs jours.

Les compositions connues ne proposent pas non plus une composition qui en plus d'une stabilité augmentée et d'un effet antimicrobien, offre une plus grande liberté pour moduler les doses lors de l'administration.

Il existe donc un réel besoin de composition aqueuse multiusage, pouvant être administrée par voie orale ou parentérale, palliant ces défauts, inconvénients et obstacles de l'art antérieur. Il existe également un besoin pour un flacon ou récipient comprenant de telles compositions permettant de maîtriser de manière extrêmement précise le dosage chez l'homme ou l'animal, de réduire les coûts et d'améliorer ainsi le traitement de manière globale.

### Exposé de l'invention

Il est du mérite de la demanderesse d'avoir développé des compositions de torasémide comprenant au moins un solvant organique tel que décrit dans la revendication 1 à un pourcentage massique supérieur ou égal à 30%. Non seulement la stabilité physique, chimique et/ou antimicrobienne de la composition est améliorée, mais celle-ci l'est dans des intervalles de pH étendus, à savoir entre 7,5 et 10, de préférence entre 8 et 9,9 et de manière encore plus préférée entre 9 et 9,9. Ainsi, les compositions selon l'invention sont stables sur une durée d'au moins 24 mois, de préférence 36 mois à une température de conservation comprise entre 25 et 30 °C. L'amélioration de ces propriétés rend la composition apte à un usage multiple (ou multiusage), à savoir une utilisation répétée de la même composition sur une période de temps s'étalant de plusieurs heures à plusieurs jours.

Les compositions selon l'invention ont en outre une efficacité de conservation antimicrobienne conforme aux pharmacopées américaine et européenne.

Ainsi, non seulement la stabilité est améliorée, mais elle l'est dans une gamme de pH étendue et la composition bénéficie en outre d'un effet antimicrobien. Ce dernier permet par exemple de faire l'économie d'un conservateur antimicrobien dans la composition de torasémide.

L'invention se rapporte ainsi à une composition aqueuse comprenant du torasémide et au moins un solvant organique tel que décrit dans la revendication 1, caractérisée en ce que la concentration massique en solvant organique peut être supérieure ou égale à 30 % par rapport à la masse totale de la composition, de préférence supérieure ou égale à 35 % et de manière encore plus préférée supérieure ou égale à 40 %. De préférence, la concentration en solvant organique peut être inférieure ou égale à 70 %.

On entend par « composition aqueuse », une composition comprenant de l'eau. Dans le cadre de l'invention une composition aqueuse comprend au moins de l'eau, un solvant organique et du torasémide.

Avantageusement, la concentration massique en torasémide peut être comprise entre 0,01 % et 5 % par rapport à la masse totale de la composition, de préférence entre 0,1 et 3 % et de manière encore plus préférée entre 0,1 et 2 %. La concentration massique en torasémide exprimé en mg.ml⁻¹ peut être comprise entre 0,1 et 50 mg.ml⁻¹, de préférence entre 1 et 30 mg.ml⁻¹ et de manière encore plus préférée entre 1 et 20 mg.ml⁻¹. La concentration massique en torasémide peut par exemple être 1, 2, 3, 4, 5, 6, 7, 8, 10, 12, 15 ou 20 mg.ml⁻¹. Avantageusement, les compositions selon l'invention sont exemptes d'impuretés. Dans le cadre de l'invention, on entend par « impuretés », des particules issues de la dégradation du torasémide, ou de la précipitation du torasémide sous forme de cristaux, des molécules de type aldéhyde ou bien encore cétone. De préférence, la concentration totale en impuretés inconnues ou connues, telles impuretés A et B (Pharmacopée Européenne) ou impuretés A et E (selon l'USP) peut être inférieure à 5 %. Les compositions selon l'invention peuvent se présenter sous la forme de solutions limpides. Dans le cadre de l'invention, on entend par « limpide » une solution qui ne comprend pas de particules visibles selon la monographie de la pharmacopée européenne.

Le au moins un solvant organique est choisi dans le groupe comprenant les alcools dérivés du propane. Par exemple, le solvant organique est choisi dans le groupe comprenant, le propylène glycol (aussi appelé propane-1,2-diol ou PG), le glycérol (aussi appelé 1,2,3-propanetriol), le propan-1,3-diol (aussi appelé PDO), le propanol (aussi appelé propan-1-ol), l'isopropanol (aussi appelé propan-2-ol) et leurs mélanges. De préférence, le solvant organique peut être choisi dans le groupe comprenant le glycérol et le propylène glycol.

Avantageusement, les compositions selon l'invention comprennent en outre un tampon physiologiquement compatible ou un agent alcalinisant. Le tampon ou agent alcalinisant peuvent par exemple être choisis dans le groupe comprenant la trométhamine (TRIS), la triéthanolamine, la diéthanolamine, la monoéthanolamine, l'hydroxyde de sodium, l'hydroxyde de potassium, l'hydroxyde d'ammonium et la méglumine. La concentration massique en tampon ou agent alcalinisant peut être comprise entre 1 et 5 % par rapport à la masse totale de la composition, de préférence entre 1 et 2%.

Avantageusement, la composition selon l'invention peut comprendre en outre un agent permettant d'ajuster la viscosité (ou épaississant). Un tel agent permettant d'ajuster la viscosité peut par exemple être un polymère synthétique hydrophile d'acide acrylique (carbomère), un polysaccharide (dérivés cellulosiques, chitosanes, amidons ou alginates), une gomme, la polyvinylpovidone, un poloxamère (pluronic), la silice hydrophile ou bien encore un poly(méth)acrylate. Le carbomère peut être choisi dans le groupe comprenant l'homopolymère d'acide 2-propénoïque tel que par exemple le Carbopol 971NF, 974, 934P ou 941. Le polysaccharide peut être choisi dans le groupe comprenant l'amidon et ses dérivés, les dérivés de cellulose tels que par exemple l'hydroxyéthylcellulose (HEC), l'éthylcellulose (EC), la carboxyméthylcellulose (CMC) et l'hydroxypropylcellulose (HPC), les dérivés de chitosane tels que par exemple la chitine déacétylée, les copolymères de glucosamine et de N-acétylglucosamine avec différents degré d'acétylation et ayant un poids moléculaire allant de 10 000 à 1000000. Les gommes peuvent être choisies dans le groupe comprenant la gomme xanthane et la gomme guar (galactomannane). On entend par « dérivé », une base conjuguée, un sel, éther, ester ou de manière plus générale, un composé qui est dérivé d'un composé similaire par une réaction chimique. La concentration massique en agent permettant d'ajuster la viscosité peut être comprise entre 0,01 et 10 % par rapport à la masse totale de la composition, de préférence entre 0,1 et 5 % et de manière encore plus préférée entre 0,1 et 2 %.

Avantageusement, la viscosité des compositions selon l'invention peut être comprise entre 0,001 et 5 Pa.s. Une telle viscosité permet d'obtenir une distribution optimale du principe actif lors de l'administration de la composition.

Avantageusement, la composition selon l'invention peut comprendre en outre un agent permettant d'ajuster l'osmolarité de la composition. Un tel agent peut par exemple être choisi parmi le sucre (glucoses), NaCl ou KCl.

Avantageusement, la composition selon l'invention peut comprendre en outre un édulcorant. L'édulcorant peut être choisi dans le groupe comprenant la saccharine (sodique), l'acésulfame de potassium, l'advantame, l'aspartame, l'érythritol et l'isomalt. La concentration massique en édulcorant peut être comprise entre 0,01 et 2 % par rapport au poids total de la composition. On entend par « édulcorant » ou « agent sucrant » au sens de l'invention, un composé ou mélange de composés ayant un goût sucré. L'ajout de ce type de composé dans la composition augmente l'appétence de cette dernière, lorsque celle-ci est destinée à être administrée par voie orale, et la rend ainsi plus facilement acceptée par le sujet, en particulier lorsque le médicament est destiné à l'animal.

Avantageusement, la composition selon l'invention peut comprendre en outre tout excipient pharmaceutiquement acceptable. La composition peut ainsi comprendre un ou plusieurs tensioactif(s), composé(s) inorganique(s) ou agent(s) isotonique(s).

Avantageusement, les compositions selon l'invention peuvent avoir un pH compris entre 7,5 et 10, de préférence compris entre 8,0 et 9,9 et de manière encore plus préférée entre 9 et 9,9 ou encore 9,3 et 9,9. Le pH des compositions peut être facilement adapté par l'homme du métier en fonction de la solubilité et de la quantité des composants de la composition, et en particulier en fonction de la solubilité et de la quantité de torasémide. Le pH des compositions selon l'invention peut être ajusté à l'aide de tout acide ou base pharmaceutiquement acceptable. Le pH peut par exemple être ajusté au moyen de NaOH, HCl, méglumine (N-méthylglucamine) ou encore trométhamine.

La stabilité améliorée des compositions selon l'invention présente également l'avantage de permettre une plus grande modulation dans les traitements, de par la diminution des contraintes habituellement associées au torasémide. Les compositions selon l'invention apportent une solution plus adaptée à chaque patient, grâce notamment à la possibilité d'ajuster plus facilement la dose distribuée à toutes les masses corporelles potentielles. Pour la première fois dans les traitements à base de torasémide, une composition répond au problème d'ajustement de dose au poids, pour cette substance qui a une marge thérapeutique étroite.

Pour la première fois également, une composition de torasémide est adaptée à un usage multiple (ou multiusage) et permet de s'affranchir des contraintes liées aux compositions à usage unique. Cela se traduit par exemple par une diminution de la quantité d'emballage, des contraintes d'utilisation plus souples ou une facilité de conservation d'une composition déjà ouverte qui serait amenée à être réutilisée.

Les compositions selon l'invention sont ainsi adaptées tant au traitement chronique (par exemple sous forme de composition orale) qu'au traitement d'urgence (par exemple sous forme de composition injectable). Le volume de liquide administré à l'homme ou à l'animal peut être ajusté en fonction de la dose thérapeutique requise et de la masse corporelle du sujet.

Avantageusement, les compositions selon l'invention peuvent être administrées par voie orale, sous-cutanée (SC), intramusculaire (IM) ou par voie intraveineuse (IV).

Selon un autre aspect, l'invention se rapporte à une composition selon l'invention pour son utilisation comme médicament, en particulier dans le traitement des signes cliniques, y compris l'œdème et l'épanchement, liés à une insuffisance cardiaque congestive, le traitement des maladies rénales et de l'hypertension.

L'invention se rapporte ainsi également à :
- une utilisation d'une composition selon l'invention pour traiter les signes cliniques, y compris l'œdème et l'épanchement, liés à une insuffisance cardiaque congestive, pour traiter les maladies rénales et l'hypertension,
- une méthode de traitement des signes cliniques, y compris l'œdème et l'épanchement, liés à une insuffisance cardiaque congestive, méthode de traitement des maladies rénales et de l'hypertension, comprenant l'administration d'une composition selon l'invention.

L'invention se rapporte aussi à une utilisation d'au moins dudit solvant organique, à une concentration massique supérieure ou égale à 30 %, de préférence supérieure ou égale à 35 % et de manière encore plus préférée supérieure ou égale à 40 %, dans une composition aqueuse comprenant du torasémide. De préférence, la concentration massique en au moins un solvant organique peut être inférieure ou égale à 70%.

L'invention comprend aussi une utilisation d'au moins dudit solvant organique, à une concentration massique supérieure ou égale à 30 %, de préférence supérieure ou égale à 35 % et de manière encore plus préférée supérieure ou égale à 40 % pour augmenter la stabilité d'une composition aqueuse comprenant du torasémide et/ou conférer des propriétés antimicrobiennes à ladite composition ; c'est-à-dire que ladite composition est stable pendant une période d'au moins 24 mois, de préférence 36 mois, à une température comprise entre 25°C et 40°C, de préférence 30°C. Par exemple, la composition est stable au moins 24 mois à une température de 30°C ou au moins 36 mois à une température de 25°C. En outre, la composition, après ouverture, de par sa stabilité physique, chimique et microbiologique, peut être conservée jusqu'à 28 jours.

L'invention concerne également un procédé de préparation des compositions selon l'invention comprenant les étapes :
(a) introduction du tampon ou de l'agent alcalinisant dans de l'eau à température ambiante et agitation pour obtenir le mélange 1,
(b) ajout du torasémide au mélange 1 et agitation pour obtenir le mélange 2,
(c) ajout progressif de l'agent permettant d'ajuster la viscosité sous agitation pour obtenir le mélange 3,
(d) éventuellement ajout d'un ou plusieurs excipient(s) pharmaceutiquement acceptable(s) et agitation jusqu'à l'obtention d'une solution limpide (mélange 4),
(e) ajout du au moins dudit solvant organique et agitation jusqu'à l'obtention d'une solution limpide (mélange 5),
(f) éventuellement ajustement du volume sous agitation,
(g) éventuellement ajustement du pH,
et obtention de la composition selon l'invention.

L'agitation peut être mécanique (agitateur magnétique) et/ou effectuée à l'aide d'une turbine.

Selon un autre aspect, l'invention concerne également un flacon ou récipient comprenant une composition selon l'invention.

Avantageusement, le flacon peut être muni d'un bouchon, d'un réducteur ou de tout autre système d'ouverture facilitant la délivrance de la composition. Un tel dispositif, dans lequel s'emboite une seringue ou autre système d'administration de la composition permet, en fonction de la graduation, d'administrer juste la dose nécessaire, efficace et adaptée à chaque traitement. Un tel dispositif permet de limiter la contamination microbienne lors de l'utilisation de la composition selon l'invention, le système d'administration (i.e. la seringue) n'entrant pas en contact avec la composition. Par exemple, lors d'un contact accidentel, les propriétés antimicrobiennes de la composition la protègent d'une éventuelle contamination.

Enfin, l'invention concerne aussi un kit comprenant :
- un flacon ou récipient comprenant une composition selon l'invention ; et
- un système d'administration de la composition.

On entend par « système d'administration de la composition » tout système permettant de prélever la composition dans le flacon ou récipient pour ensuite l'administrer par voie orale, SC, IM ou IV, tel que par exemple un compte-goutte, un bouchon doseur ou une seringue.

D'autres avantages pourront encore apparaître à l'homme du métier à la lecture des exemples ci-dessous.

### Brève description des figures

- La figure 1 représente une courbe de solubilité du torasémide (en mg.ml⁻¹) en fonction du pH.
- La figure 2 représente un bouchon et un système d'administration de la composition (seringue).

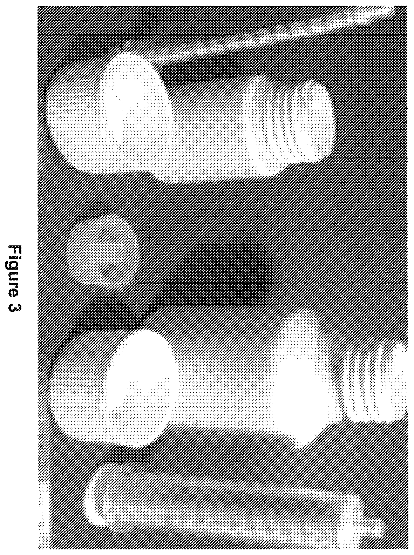

- La figure 3 représente deux exemples de kits selon l'invention (gauche et droite) comprenant un flacon muni d'un bouchon et d'un système d'administration (seringue) de la composition et un réducteur (centre).

### EXEMPLES

### Exemple 1 : Préparation d'une composition selon l'invention

### Préparation d'un mélange 1 :

On introduit de l'eau et la trométhamine (tampon) à température ambiante (18-25°C) sous agitation à l'aide d'une turbine défloculeuse (vitesse : 200-600 RPM, durée : environ 15 min).

### Préparation d'un mélange 2 :

On ajoute le torasémide au mélange 1, sous agitation à l'aide d'une turbine défloculeuse (vitesse : 200-600 RPM, durée : environ 15 min).

### Préparation d'un mélange 3 :

On ajoute progressivement le polymère au mélange 2, sous agitation à l'aide d'une turbine défloculeuse et d'une agitation mécanique (vitesse : 200-600 RPM, durée : environ 15 min).

### Préparation d'un mélange 4 :

On ajoute le reste des excipients (en dehors du solvant organique) au mélange 3, tout en maintenant l'agitation jusqu'à l'obtention d'une solution limpide.

### Préparation d'un mélange 5 :

On ajoute ensuite le solvant organique (propylène glycol) au mélange 4, tout en maintenant l'agitation jusqu'à l'obtention d'une solution limpide.

On ajuste éventuellement le volume avec de l'eau sous agitation magnétique, pendant une durée minimum de 15 min.

On ajuste éventuellement le pH à la valeur désirée.

On obtient la composition sous forme de liquide incolore et peu visqueux.

Les compositions dans le tableau 1 ci-dessous sont préparées selon le protocole ci-dessus, avec les quantités indiquées dans le tableau.

**Tableau 1 : compositions, exemple comparatif et selon l'invention.**

| **Composition n°** | **Comparatif 1** | **Composition 1** | **Composition 2** |
|---|---|---|---|
| Torasémide | 0,30% | 0,20% | 0,20% |
| Trométhamine (TRIS) | | 1,50% | 1,50% |
| Méglumine | 1,00% | | |
| PEG 400 | 10,00% | | |
| Propylène glycol | | 40,00% | 40,00% |
| Carbopol 971 P | | 0,20% | |
| Natrosol 250G Pharm | | | 1,00% |
| Saccharine sodique | | 0,20% | |
| Acide chlorhydrique | QSP pH 8,0-10 | QSP pH 9,3-9,7 | QSP pH 9,3-9,7 |
| Eau ppi | QSP 100% | QSP 100% | QSP 100% |

| | | | |
|---|---|---|---|
| QSP = quantité suffisante pour. Eau ppi = eau pour injection. | | | |

### Exemple 2 : Stabilité optimisée des compositions selon l'invention

La stabilité des compostions 1 et 2 a été évaluée dans des conditions de stockage stressantes accélérées : à une température de 40°C, à un taux d'humidité relative de 75% et pendant un durée de T = 2 mois.

Pour chacune des compositions testées, le taux d'impuretés est mesuré selon la méthode correspondant au principe actif, décrite dans les monographies de la pharmacopée européenne ou américaine.

Les résultats sont compilés dans le tableau 2 ci-dessous :

**Tableau 2 : essais de stabilité des compositions 1 et 2.**

| **Composition n°** | **Comparatif 1** | **Composition 1** | **Composition 2** |
|---|---|---|---|
| Aspect macroscopique | trouble | limpide | limpide |
| teneur en torasémide (par dosage, 95-105%) | 100,30 | 99,10 | 100,00 |
| Chaque impureté inconnue (≤0,3%) | **0,31** | **0,02** | **0,01** |
| Impuretés inconnues totales (≤1,0%) | **0,41** | **0,07** | **0,04** |

On entend par « impureté inconnue », les impuretés, produits de dégradation et impuretés chimiques classées « B » et « A » dans la pharmacopée européenne et « A » et « E » dans la pharmacopée américaine (USP).

Les résultats obtenus démontrent que les compositions selon l'invention sont plus stables que les compositions de l'état de la technique. Les compositions selon l'invention sont ainsi conformes aux critères exigés par les pharmacopées américaine et européenne en termes de stabilité.

### Exemple 3 : Propriétés antimicrobiennes des compositions selon l'invention

L'évaluation de l'efficacité antimicrobienne a été réalisée selon la monographie européenne.

Les compositions suivantes (A à I selon l'invention et CE1 à CE7 contre-exemples) ont été préparées selon le mode opératoire de l'exemple 1.

**Tableau 3 : Efficacité de conservation antimicrobienne de différents lots avec 0.2 % de carbomère et avec différentes quantités de propylène glycol. NC = non conforme, C = conforme.**

| **Ingrédients** | **Composition centésimale (en %m/m)** | | |
|---|---|---|---|
| | **CE1 (0% PG)** | **CE2 (20% PG)** | **A (40% PG)** |
| Torasémide | 0,20 | 0,20 | 0,20 |
| Trométhamine | 1,50 | 1,50 | 1,50 |
| Carbomère | 0,20 | 0,20 | 0,20 |
| **Propylène glycol** | **0,00** | **20,00** | **40,00** |
| Eau purifiée q.s.p m/m | 98,10 | 78,10 | 58,10 |
| **Total (% w/w)** | 100,00 | 100,00 | 100,00 |
| | | | |

| **Efficacité de conservation antimicrobienne (Ph Eur)** | NC | NC | **C** |
|---|---|---|---|
| S. *aureus* | **C** | **C** | **C** |
| *E. coli* | **C** | **C** | c |
| *Ps. aeruginosa* | **C** | **C** | **C** |
| C. *albicans* | **C** | **C** | **C** |
| *A. brasiliensis* | NC | NC | **C** |

**Tableau 4 : Efficacité de conservation antimicrobienne de différents lots avec 1 % de HEC et avec des quantités différentes de propylène glycol.**

| **Ingrédients** | **Composition centésimale (en %m/m)** | | |
|---|---|---|---|
| | **CE3 (0% PG)** | **CE4 (20% PG)** | **B (40% PG)** |
| Torasémide | 0.20 | 0.20 | 0.20 |
| Trométhamine | 1.50 | 1.50 | 1.50 |
| Hydroxyéthyl cellulose | 1.00 | 1.00 | 1.00 |
| **Propylène glycol** | **0.00** | **20.00** | **40.00** |
| Eau purifiée q.s.p m/m | 97.30 | 77.30 | 57.30 |
| **Total (%w/w**) | 100.00 | 100.00 | 100.00 |
| | | | |

| **Efficacité de conservation antimicrobienne (Ph Eur)** | NC | NC | **C** |
|---|---|---|---|
| S. *aureus* | NC | **C** | **C** |
| *E. coli* | **C** | **C** | **C** |
| *Ps. aeruginosa* | **C** | NC | **C** |
| C. *albicans* | NC | **C** | **C** |
| *A. brasiliensis* | NC | NC | **C** |

| | | | |
|---|---|---|---|
| NC = non conforme, C = conforme. | | | |

**Tableau 5 : Efficacité de conservation antimicrobienne de différents lots avec 0,3 % de gomme xanthane et avec les quantités différentes de propylène glycol. NC = non conforme, C = conforme.**

| **Ingrédients** | **Composition centésimale (en %m/m)** | | |
|---|---|---|---|
| | **CE5** | **C (35% PG)** | **D (45% PG)** |
| Torasémide | 0,20 | 0,20 | 0,20 |
| Trométhamine | 1,50 | 1,50 | 1,50 |
| Gomme xanthane | 0,30 | 0,30 | 0,30 |
| **Propylène glycol** | **0,00** | **35,00** | **45,00** |
| Eau purifiée q.s.p m/m | 98,00 | 63,00 | 53,00 |
| **Total (%w/w**) | 100,00 | 100,00 | 100,00 |
| | | | |

| **Efficacité de conservation antimicrobienne (Ph Eur)** | NC | **C** | **C** |
|---|---|---|---|
| S. *aureus* | NC | **C** | **C** |
| *E. coli* | **C** | **C** | c |
| *Ps. aeruginosa* | NC | **C** | **C** |
| C. *albicans* | NC | **C** | **C** |
| *A. brasiliensis* | NC | **C** | **C** |

| **Efficacité de conservation antimicrobienne (USP)** | NC | **C** | **C** |
|---|---|---|---|
| S. *aureus* | **C** | **C** | **C** |
| *E. coli* | **C** | **C** | c |
| *Ps. aeruginosa* | NC | **C** | **C** |
| C. *albicans* | **C** | **C** | **C** |
| *A. brasiliensis* | **C** | **C** | **C** |

**Tableau 6 : Evaluation de l'efficacité antimicrobienne du Propylène Glycol en fonction de sa teneur avec 0,15% de Carbopol 971P. Elles diffèrent entre elles, par la teneur en propylène glycol : 0, 30, 45 et 60%m/m. NC = non conforme, C = conforme.**

| **Ingrédients** | **Composition centésimale (en %m/m)** | | | |
|---|---|---|---|---|
| | **CE6** | **E** | **F** | **G** |
| Torasémide | 0,20 | 0,20 | 0,20 | 0,20 |
| Trométhamine | 1,50 | 1,50 | 1,50 | 1,50 |
| **Propylène Glycol** | / | **30,00** | **45,00** | **60,00** |
| Carbopol 971P | 0,15 | 0.15 | 0,15 | 0,15 |
| Eau purifiée q.s.p m/m | q.s.p 100,00 | q.s.p 100.00 | q.s.p 100,00 | q.s.p 100,00 |
| | | | | |

| **Efficacité de conservation antimicrobienne (Ph Eur)** | NC | **C** | **C** | C |
|---|---|---|---|---|
| S. *aureus* | **C** | **C** | C | C |
| *E. coli* | **C** | **C** | C | C |
| *Ps. aeruginosa* | **C** | **C** | **C** | C |
| C. *albicans* | **C** | **C** | C | C |
| *A. brasiliensis* | NC | **C** | **C** | **C** |

**Tableau 7 : Evaluation de l'efficacité antimicrobienne du Propylène Glycol en fonction de sa teneur avec 0,15% de gomme xanthane. Elles diffèrent entre elles, par la teneur en propylène glycol : 0, 45 et 60%m/m. NC = non conforme, C = conforme.**

| **Ingrédients** | **Composition centésimale (en %m/m)** | | |
|---|---|---|---|
| | **CE7** | **H** | **I** |
| Torasémide | 0,20 | 0,20 | 0,20 |
| Trométhamine | 1,50 | 1,50 | 1,50 |
| **Propylène Glycol** | / | **45,00** | **60,00** |
| Gomme Xanthane | 0,20 | 0,20 | 0,20 |
| Eau purifiée q.s.p m/m | q.s.p 100,00 | q.s.p 100,00 | q.s.p 100,00 |
| | | | |

| **Efficacité de conservation antimicrobienne (Ph Eur)** | NC | **C** | **C** |
|---|---|---|---|
| S. *aureus* | NC | **C** | **C** |
| *E. coli* | **C** | **C** | **C** |
| *Ps. aeruginosa* | NC | **C** | **C** |
| C. *albicans* | NC | **C** | **C** |
| *A. brasiliensis* | NC | **C** | **C** |

Les compositions comprenant une teneur supérieur ou égale à 30 % de propylène glycol ont permis d'obtenir une efficacité de conservation antimicrobienne conforme à la pharmacopée européenne, ladite pharmacopée ayant les critères les plus stricts (intervalles de tolérance plus resserrés) en termes d'efficacité de conservation antimicrobienne, contrairement aux compositions comprenant moins de 30 % de solvant organique (i.e. propylène glycol).

### Exemple 4 : Comparaison de stabilité de compositions comprenant furosémide ou torasémide

Les compositions CE-A à CE-F sont préparées selon le mode opératoire suivant :
1) ajout de l'éthanol dans le propylène glycol et homogénéisation ;
2) ajout du tampon citrate dans le mélange obtenu à l'étape 1 ;
3) ajout du principe actif (furosémide ou torasémide) dans le mélange obtenu à l'étape 2 ;
4) ajout de HPC (hydroxypropyl cellulose)) dans le mélange obtenu à l'étape 3.

Les compostions CE-A à CE-F ont toutes un pH compris entre 6,0 et 6,6.

**Tableau 7 : Composition et aspect des compositions CE-A à CE-F. L = solution limpide, S = suspension, P = particules.**

| Formule | CE-A (% w/w) | CE-B (% w/w) | CE-C (% w/w) | CE-D (% w/w) | CE-E (% w/w) | CE-F (% w/w) |
|---|---|---|---|---|---|---|
| Furosémide | 0,125 | | | | | |
| Torasémide | | 0,200 | 0,200 | 1,000 | 3,000 | 3,000 |
| Propylène glycol | 48,438 | 48,363 | 60,000 | 48,363 | 24,313 | 48,363 |
| Ethanol absolu | 38,750 | 20,000 | 20,000 | 38,750 | 60,000 | 38,750 |
| HPC | 3,000 | 3,000 | 3,000 | 3,000 | 3,000 | 3,000 |
| Tampon citrate | 9,688 | 28,438 | 16,800 | 8,888 | 9,688 | 6,888 |
| TOTAL | 100,001 | 100,000 | 100,000 | 100,001 | 100,001 | 100,001 |
| Aspect | L | S | P | S | S | S |

Ces résultats montrent que furosémide et torasémide ne sont pas simplement interchangeables.

### Liste des références

[1] Peddle et al (J. Vet. Cardiology, 2012)
[2] US 4,861,786 A

## Revendications

1. Composition aqueuse comprenant du torasémide, un tampon ou un agent alcalinisant et au moins un solvant organique choisi dans le groupe comprenant les alcools dérivés du propane, de préférence choisi parmi le propylène glycol, le glycérol, le propan-1,3-diol, le propanol, l'isopropanol et leurs mélanges, **caractérisée en ce que** la concentration massique en solvant organique est supérieure ou égale à 30 % par rapport à la masse totale de la composition, de préférence supérieure ou égale à 35% et de manière encore plus préférée supérieure ou égale à 40 %.

2. Composition selon la revendication 1, dans laquelle la concentration massique en torasémide est comprise entre 0,01 % et 5 % par rapport à la masse totale de la composition, de préférence entre 0,1 et 3 % et de manière encore plus préférée entre 0,1 et 2 %.

3. Composition selon l'une quelconque des revendications précédentes, dans laquelle le tampon ou agent alcalinisant est choisi dans le groupe comprenant la trométhamine (TRIS), la triéthanolamine, la diéthanolamine, la monoéthanolamine, l'hydroxyde de sodium, l'hydroxyde de potassium, l'hydroxyde d'ammonium et la méglumine, la concentration massique en tampon ou en agent alcalinisant étant de préférence comprise entre 1 et 5 % par rapport à la masse totale de la composition.

4. Composition selon l'une quelconque des revendications précédentes, comprenant en outre un agent permettant d'ajuster la viscosité, choisi dans le groupe comprenant les polymères synthétiques hydrophiles d'acide acrylique, les polysaccharides, les gommes, la polyvinylpovidone, les poloxamères, la silice hydrophile et les poly(méth)acrylates, la concentration massique en agent permettant d'ajuster la viscosité étant de préférence comprise entre 0,01 et 10 % par rapport à la masse totale de la composition.

5. Composition selon l'une quelconque des revendications précédentes, comprenant en outre un édulcorant.

6. Composition selon la revendication précédente, dans laquelle la viscosité est comprise entre 0,001 et 5 Pa.s.

7. Composition selon l'une quelconque des revendications précédentes, comprenant en outre un ou plusieurs excipient(s) pharmaceutiquement acceptable(s).

8. Composition selon l'une quelconque des revendications précédentes, dans laquelle le pH est compris entre 7,5 et 10, de préférence compris entre 8,0 et 9,9 et de manière encore plus préférée entre 9 et 9,9 ou encore 9,3 et 9,9.

9. Composition selon l'une quelconque des revendications 1 à 8, pour son utilisation comme médicament, de préférence destinée à être utilisée dans le traitement des signes cliniques, y compris l'œdème et l'épanchement, liés à une insuffisance cardiaque congestive, le traitement des maladies rénales et de l'hypertension.

10. Utilisation d'au moins un solvant organique choisi dans le groupe comprenant les alcools dérivés du propane, de préférence choisi parmi le propylène glycol, le glycérol, le propan-1,3-diol, le propanol, l'isopropanol et leurs mélanges, à une concentration massique supérieure ou égale à 30 %, de préférence supérieure à 35 % et de manière encore plus préférée supérieure à 40 %, dans une composition aqueuse comprenant du torasémide.

11. Utilisation d'au moins un solvant organique choisi dans le groupe comprenant les alcools dérivés du propane, de préférence choisi parmi le propylène glycol, le glycérol, le propan-1,3-diol, le propanol, l'isopropanol et leurs mélanges, à une concentration massique supérieure ou égale à 30 %, de préférence supérieure à 35 % et de manière encore plus préférée supérieure à 40 %, pour augmenter la stabilité d'une composition aqueuse comprenant du torasémide et/ou conférer des propriétés antimicrobiennes à ladite composition.

12. Procédé de préparation d'une composition selon l'une quelconque des revendications 1 à 9, comprenant les étapes, éventuellement sous atmosphère inerte, de :
(a) introduction du tampon ou de l'agent alcalinisant dans de l'eau à température ambiante et agitation pour obtenir le mélange 1,
(b) ajout du torasémide au mélange 1 et agitation pour obtenir le mélange 2,
(c) ajout progressif de l'agent permettant d'ajuster la viscosité, sous agitation, pour obtenir le mélange 3,
(d) éventuellement, ajout d'un ou plusieurs excipient(s) pharmaceutiquement acceptable(s), et agitation jusqu'à l'obtention d'une solution limpide, pour obtenir le mélange 4,
(e) ajout du au moins un solvant organique et agitation jusqu'à l'obtention d'une solution limpide, pour obtenir le mélange 5,
(f) éventuellement, ajustement du volume sous agitation,
(g) éventuellement, ajustement du pH,
et obtention de la composition selon l'invention.

13. Flacon ou récipient comprenant une composition selon l'une quelconque des revendications 1 à 9.

14. Kit comprenant :
- un flacon ou récipient selon la revendication précédente ; et
- un système d'administration de la composition.

## Patentansprüche

1. Wässrige Zusammensetzung, die Torasemid, einen Puffer oder ein Alkalisierungsmittel und mindestens ein organisches Lösungsmittel umfasst, das aus der Gruppe ausgewählt ist, die von Propan abgeleitete Alkohole umfasst, die vorzugsweise aus Propylenglykol, Glycerin, Propan-1,3-diol, Propanol, Isopropanol und deren Mischungen ausgewählt sind, **dadurch gekennzeichnet, dass** die Konzentration des organischen Lösungsmittels, bezogen auf das Gewicht, größer oder gleich 30 %, bezogen auf das Gesamtgewicht der Zusammensetzung, ist, vorzugsweise größer oder gleich 35 % und noch bevorzugter größer oder gleich 40 %.

2. Zusammensetzung nach Anspruch 1, wobei die Gewichtskonzentration von Torasemid zwischen 0,01 % und 5 %, bezogen auf das Gesamtgewicht der Zusammensetzung, vorzugsweise zwischen 0,1 und 3 % und noch bevorzugter zwischen 0,1 und 2 % liegt.

3. Zusammensetzung nach einem der vorhergehenden Ansprüche, wobei der Puffer oder das Alkalisierungsmittel aus der Gruppe ausgewählt ist, die Tromethamin (TRIS), Triethanolamin, Diethanolamin, Monoethanolamin, Natriumhydroxid, Kaliumhydroxid, Ammoniumhydroxid und Meglumin umfasst, wobei die Konzentration des Puffers oder des Alkalisierungsmittels in Gewichtsprozent vorzugsweise zwischen 1 und 5 %, bezogen auf das Gesamtgewicht der Zusammensetzung, liegt.

4. Zusammensetzung nach einem der vorhergehenden Ansprüche, die ferner ein Mittel zur Einstellung der Viskosität enthält, das aus der Gruppe ausgewählt ist, die synthetische hydrophile Polymere der Acrylsäure, Polysaccharide, Gummis, Polyvinylpovidon, Poloxamere, hydrophiles Siliciumdioxid und Poly(meth)acrylate umfasst, wobei die Gewichtskonzentration des Rheologiemodifikators vorzugsweise zwischen 0,01 und 10 %, bezogen auf das Gesamtgewicht der Zusammensetzung, liegt.

5. Die in einem der vorhergehenden Ansprüche beanspruchte Zusammensetzung enthält außerdem einen Süßstoff.

6. Die Zusammensetzung nach dem vorhergehenden Anspruch, wobei die Viskosität zwischen 0,001 und 5 Pa.s liegt.

7. Die Zusammensetzung nach einem der vorangehenden Ansprüche, die ferner einen oder mehrere pharmazeutisch annehmbare(n) Hilfsstoff(e) enthält.

8. Zusammensetzung nach einem der vorangehenden Ansprüche, wobei der pH-Wert zwischen 7,5 und 10, vorzugsweise zwischen 8,0 und 9,9 und noch bevorzugter zwischen 9 und 9,9 oder auch 9,3 und 9,9 liegt.

9. Zusammensetzung nach einem der Ansprüche 1 bis 8 zur Verwendung als Arzneimittel, vorzugsweise zur Verwendung bei der Behandlung von klinischen Symptomen, einschließlich Ödemen und Flüssigkeitsansammlungen, die mit kongestiver Herzinsuffizienz, Behandlung von Nierenerkrankungen und Bluthochdruck einhergehen.

10. Verwendung mindestens eines organischen Lösungsmittels, ausgewählt aus der Gruppe der von Propan abgeleiteten Alkohole, vorzugsweise ausgewählt aus Propylenglykol, Glycerin, Propan-1,3-diol, Propanol, Isopropanol und deren Mischungen, in einer Gewichtskonzentration von mehr als oder gleich 30 %, vorzugsweise mehr als 35 % und noch bevorzugter mehr als 40 %, in einer wässrigen Zusammensetzung, die Torasemid enthält.

11. Verwendung mindestens eines organischen Lösungsmittels, ausgewählt aus der Gruppe der von Propan abgeleiteten Alkohole, vorzugsweise ausgewählt aus Propylenglykol, Glycerin, Propan-1,3-diol, Propanol, Isopropanol und deren Mischungen, in einer Gewichtskonzentration von mehr als oder gleich 30 %, vorzugsweise mehr als 35 % und noch bevorzugter mehr als 40 %, zur Erhöhung der Stabilität einer wässrigen Zusammensetzung, die Torasemid enthält, und/oder zur Ausstattung der Zusammensetzung mit antimikrobiellen Eigenschaften.

12. Verfahren zur Herstellung einer Zusammensetzung nach einem der Ansprüche 1 bis 9, umfassend die Schritte, gegebenenfalls unter einer inerten Atmosphäre, von:
(a) Zugabe des Puffers oder des Alkalisierungsmittels zu Wasser bei Raumtemperatur und Rühren, um Mischung 1 zu erhalten,
(b) Zugabe von Torasemid zu Mischung 1 und Rühren, um Mischung 2 zu erhalten,
(c) schrittweise Zugabe des Rheologiemodifikators unter Rühren, um Mischung 3 zu erhalten,
(d) gegebenenfalls Zugabe eines oder mehrerer pharmazeutisch annehmbarer Hilfsstoffe und Rühren, bis eine klare Lösung erhalten wird, um Mischung 4 zu erhalten,
(e) Zugabe mindestens eines organischen Lösungsmittels und Rühren, bis eine klare Lösung erhalten wird, um Mischung 5 zu erhalten,
(f) gegebenenfalls Einstellung des Volumens unter Rühren,
(g) gegebenenfalls Einstellung des pH-Werts,
und die erfindungsgemäße Zusammensetzung zu erhalten.

13. Flasche oder Behälter mit einer Zusammensetzung nach einem der Ansprüche 1 bis 9.

14. Kit bestehend aus:
- eine Flasche oder ein Behältnis gemäß dem vorhergehenden Anspruch; und
- ein System zur Verabreichung der Zusammensetzung.

## Claims

1. An aqueous composition comprising torasemide, a buffer or an alkalizing agent and at least one organic solvent selected from the group comprising alcohols derived from propane, preferably selected from propylene glycol, glycerol, propane-1,3-diol, propanol, isopropanol and mixtures thereof, **characterized in that** the concentration of organic solvent by weight is greater than or equal to 30% relative to the total weight of the composition, preferably greater than or equal to 35% and even more preferably greater than or equal to 40%.

2. The composition as claimed in claim 1, wherein the concentration of torasemide by weight is between 0.01 % and 5% relative to the total weight of the composition, preferably between 0.1 and 3% and even more preferably between 0.1 and 2%.

3. The composition as claimed in any one of the preceding claims, wherein the buffer or alkalizing agent is selected from the group comprising tromethamine (TRIS), triethanolamine, diethanolamine, monoethanolamine, sodium hydroxide, potassium hydroxide, ammonium hydroxide and meglumine, the concentration of buffer or of alkalizing agent by weight preferably being between 1 and 5% relative to the total weight of the composition.

4. The composition as claimed in any one of the preceding claims, further comprising a rheology modifier, selected from the group comprising synthetic hydrophilic polymers of acrylic acid, polysaccharides, gums, polyvinyl povidone, poloxamers, hydrophilic silica and poly(meth)acrylates, the concentration of rheology modifier by weight preferably being between 0.01 and 10% relative to the total weight of the composition.

5. The composition as claimed in any one of the preceding claims, further comprising a sweetener.

6. The composition as claimed in the preceding claim, wherein the viscosity is between 0.001 and 5 Pa.s.

7. The composition as claimed in any one of the preceding claims, further comprising one or more pharmaceutically acceptable excipient(s).

8. The composition as claimed in any one of the preceding claims, wherein the pH is between 7.5 and 10, preferably between 8.0 and 9.9 and even more preferably between 9 and 9.9 or else 9.3 and 9.9.

9. The composition as claimed in any one of claims 1 to 8, for use as a drug, preferably intended to be used in the treatment of clinical signs, including edema and fluid collection, associated with congestive heart failure, treatment of kidney diseases and hypertension.

10. The use of at least one organic solvent selected from the group comprising alcohols derived from propane, preferably selected from propylene glycol, glycerol, propane-1,3-diol, propanol, isopropanol and mixtures thereof, at a concentration by weight greater than or equal to 30%, preferably above 35% and even more preferably above 40%, in an aqueous composition comprising torasemide.

11. The use of at least one organic solvent selected from the group comprising alcohols derived from propane, preferably selected from propylene glycol, glycerol, propane-1,3-diol, propanol, isopropanol and mixtures thereof, at a concentration by weight greater than or equal to 30%, preferably above 35% and even more preferably above 40%, for increasing the stability of an aqueous composition comprising torasemide and/or endowing said composition with antimicrobial properties.

12. A method for preparing a composition as claimed in any one of claims 1 to 9, comprising the steps, optionally under an inert atmosphere, of:
(a) adding the buffer or the alkalizing agent to water at room temperature and stirring to obtain mixture 1,
(b) adding torasemide to mixture 1 and stirring to obtain mixture 2,
(c) gradually adding the rheology modifier, with stirring, to obtain mixture 3,
(d) optionally, adding one or more pharmaceutically acceptable excipient(s), and stirring until a clear solution is obtained, to obtain mixture 4,
(e) adding at least one organic solvent and stirring until a clear solution is obtained, to obtain mixture 5,
(f) optionally, adjusting the volume, with stirring,
(g) optionally, adjusting the pH,
and obtaining the composition according to the invention.

13. Bottle or container comprising a composition as claimed in any one of claims 1 to 9.

14. Kit comprising:
- a bottle or container as claimed in the preceding claim; and
- a system for administering the composition.
